# EUROPEAN PATENT SPECIFICATION

(11) **EP 1 872 129 B1**
(45) Date of publication and mention of the grant of the patent: **28.07.2010**
(21) Application number: 06784359.9
(22) Date of filing: 12.04.2006
(51) Int. Cl.: G01N 33/543

(54) **PROTEOLIPID MEMBRANE AND LIPID MEMBRANE BIOSENSOR**
PROTEOLIPIDMEMBRAN UND LIPIDMEMBRANEN-BIOSENSOR
BIODETECTEUR DE MEMBRANE PROTEOLIPIDIQUE ET DE MEMBRANE LIPIDIQUE

(30) Priority: 12.04.2005 US 670524 P
(43) Date of publication of application: 02.01.2008
(73) Proprietor: SRU Biosystems, Inc., Woburn, MA 01801 (US)
(72) Inventor: BAIRD, Cheryl, L., Pasco WA 99301 (US); WANG, Guo, Bin, Malden, MA 02148 (US); LAING, Lance, Belmont, MA 02478 (US); JOGIKALMATH, Gangadhar, Cambridge, MA-02140 (US); GERSTENMAIER, John, Cleveland Heights OH 44121 (US)
(74) Representative: Savic, Bojan
(86) International application number: PCT/US2006/013926
(87) International publication number: WO 2006/127167

(56) References cited:
- WO-A-96/38726
- US-A1- 2003 068 657
- US-B1- 6 346 376
- CUNNINGHAM B ET AL: "A plastic colorimetric resonant optical biosensor for multiparallel detection of label-free biochemical interactions" SENSORS AND ACTUATORS B, ELSEVIER SEQUOIA S.A., LAUSANNE, CH, vol. 85, no. 3, 25 July 2002 (2002-07-25), pages 219-226, XP004368813 ISSN: 0925-4005
- CUNNINGHAM B ET AL: "Colorimetric resonant reflection as a direct biochemical assay technique" SENSORS AND ACTUATORS B, ELSEVIER SEQUOIA S.A., LAUSANNE, CH, vol. 81, no. 2-3, 5 January 2002 (2002-01-05), pages 316-328, XP004329915 ISSN: 0925-4005

## Description

### BACKGROUND OF THE INVENTION

It can be difficult to assay lipids with conventional biosensors such as Biacore's X, 2000, 3000, T100, S51 and C systems or label-requiring techniques such as fluorescence-based approaches because labels do not work well in non-polar environments having various issues with quenching or excitation as known to those practiced in the art. In addition, labels often perturb systems they are used to study lipids in unpredictable and more importantly in unwanted ways. Flow based systems such as Biacore perturb the structurally fragile environment required to maintain and make measurements in non-polar environments. In addition, SPR flow formats provide only very low sample throughput which can add considerably to the time to develop proper environments, allow proper protein attachment, assembly & folding, and increase the amount of time to finally test for any interactions with a protein should it become properly attached and folded in the flow environment.

WO 96/38726 describes a grating-based waveguide biosensor having a first layer which is covalently attached a second linking layer, to which are attached third and fourth proximal phospholipid and distal lipid layers.

US 2003/068657 describes a biosensor comprising an optical grating coated with a high refractive index dielectric film and a lipid or phosphor lipid layer which may serve as the linker agent for the immobilization of specific binding substances.

US 6,346,376 describes an optical biosensor unit comprising an integrated transducer chip on which at least a layer of sensing biomolecules is bonded.

### SUMMARY OF THE INVENTION

One embodiment of the invention provides a colorimetric resonant biosensor or grating-based waveguide biosensor, wherein the surface of the biosensor is titanium oxide, titanium dioxide, or titanium phosphate, and wherein a lipid bilayer or a lipid monolayer are immobilized on the titanium oxide or titanium phosphate surface. The lipid bilayer or lipid monolayer can have no label. The biosensor can be incorporated into the bottom of a microtiter plate or can be in a microarray format. The biosensor can incorporated into the bottom of a microtiter plate, wherein each well of the microtiter plate is about about 5 mm² to about 50 mm². The titanium oxide, titanium dioxide, or titanium phosphate surface is coated with silane to form a titanium-silane or a titanium phosphate-silane surface. The biosensor can be further coated with one or more surfactants. The titanium-silane surface or titanium phosphate-silane surface can be coated with block copolymers of polyethylene oxide and polypropylene oxide in the form of PEO(a)-PPO(b)-PEO(a).

A "lipid layer" as defined in the present invention is either a lipid monolayer or a lipid bilayer.

Another embodiment of the invention provides a method of analyzing a chemical or physical interaction in a lipid layer, wherein the lipid layer is immobilized to a colorimetric resonant biosensor or a grating-based waveguide biosensor. The method comprises contacting the lipid layer with a species and analyzing the interaction of the lipid layer and the species by (a) detecting a maxima in reflected wavelength or a minima in transmitted wavelength of light used to illuminate the biosensor, wherein if the wavelength of light is shifted the species has interacted with the lipid layer; or (b) detecting a change in refractive index of light used to illuminate the biosensor, wherein a change in refractive index indicated that the species has interacted with the lipid layer. About 300 or more samples can be analyzed in about ten minutes or less. The lipid layer can be contacted with a species under static conditions. The interaction of the lipid layer and the species is analyzed under static conditions. The lipid layer and the species can be label-free.

The present invention provides biosensors in microtiter plate-based or microarray formats that allow for lipid and lipid-based assays with much higher sample number/readings per unit time. Currently, a typical assay for a single binding interaction event with a liposome SPR sensor on a single instrument takes 20-30 minutes. *See, e.g.,* Baird et al., Analyt. Biochem. 2002 310:93-99. In this amount of time, the present invention could make readings for 4 to 6 x 384-well sample biosensor plates on a single instrument. Advantageously, labels are not required for detection in the instant invention.

### BRIEF DESCRIPTION OF THE DRAWINGS

Figure 1 shows lipid applications for a colorimetric resonant biosensor, e.g., the SRU Biosystems BIND® biosensor.
Figure 2 shows a process to make liposomes.
Figure 3 shows that repel silane is a more effective hydrophobic surface treatment than a hexane wash.
Figure 4 shows streptavidin binding after PPL and aldehyde treatment.
Figure 5 shows Poly-Phe-Lys vs. Poly-Lys attachment after repel silane treatment.
Figure 6 shows the results of optimizing the hydrophobic coating to improve the capture of the model protein PPL.
Figure 7 shows a water contact angle measurement for a repel silane treated biosensor.
Figure 8 demonstrates higher amounts of PLURONIC® surfactant binding due to favorable interaction between the hydrophobic block of the surfactant (polypropylene oxide) to the hydrophobic repel surface. N=24 for PLURONIC® wells and N=8 for control wells.
Figure 9 shows the reduction in streptavidin binding to bare TiO as a function of molecular weight of the PLURONIC® surfactants.
Figure 10 shows that a higher density of PLURONIC® adsorbed surface reduces streptavidin binding to the underlying hydrophobic TiO surface. Control surfaces include bare TiO and repel modified TiO. N=24 for PLURONIC® wells and N=8 for control wells.
Figure 11 shows streptavidin binding to various PLURONIC® modified surfaces.
Figure 12 shows a BIND Imager^{™} image demonstrating wells with different surface densities of streptavidin on PLURONIC® coated surfaces.
Figure 13 shows that a biosensor surface can be assembled with stable detergent absorption.
Figure 14 shows a BIND BIOSENSOR® signal for aldehyde modification of a silane modified TiO and a TiO/SiO2 surface. N=1 plate (96 wells).
Figure 15 shows an improvement in aldehyde modification as evidenced by higher BIND BIOSENSOR® PWV shift for TIP modified silane surfaces. The results are repeatable. N=1 plate (96 wells).
Figure 16 shows the permanency of the TiP layer when the samples treated with phosphoric acid were dried at 18h at 80°C.

### DETAILED DESCRIPTION OF THE INVENTION

Stenlund *et al.,* shows the difficulty of trying to create a suitable environment for active membrane bound proteins in commercially available SPR devices. See, Analyt. Biochem. 2003, 316:243-250. These devices typically are flow-based devices, which complicates the process. Stenlund showed that the proper composition of hydrophobic material and detergent was critical, needed to be attained, was likely to be different for different proteins, and that the composition could only be attained empirically. Economically, speaking of both time and money, this type of development for scientists within the constraints of commercial enterprises can only be accomplished with a device like a colorimetric resonant biosensor or a grating-based waveguide that provides for large surface areas in a multi-well microtiter plate-based or microarray slide-based format.

Furthermore, the SPR types of devices have very small dimensions (defined significantly by the technical and economic aspects of sampling space) that are likely to become clogged by the sub-optimal "turbid" compositions of hydrophobic material and detergents. This type of clogging is not possible with a colorimetric resonant biosensor slide or microtiter plate-based device or a grating-based waveguide. Turbidity in a colorimetric resonant biosensor also does not have the high cost of failure as do the current SPR devices. Furthermore, as Stenlund demonstrated, the most likely active proteins with their approach were those that were inadvertently active following incomplete solubilization of the original protein-containing cellular components. A colorimetric resonant biosensor or grating-based waveguide device would provide sufficient and practical surface for the direct application of the weakly solubilized but active protein-containing cellular components as is, leading to a higher proportion of active protein by yet another route. This route is not available to the Stenlund approach as the weakly solubilized cellular components would most likely clog the device they employed and provide insufficient surface area for the proper attachment of the material. Furthermore, the proper attachment and folding of membrane bound proteins removed from their native environments has been shown to have a fairly significant time factor (see Cantor and Schimmel, parts 1-3 Biophysical Chemistry - The behavior and study of biological molecules, W.H. Freeman and Company, New York, copyright 1980; specifically Chapter 25 pp1327-1371: Introduction to Membrane Equilibria and to Bilayers), a factor that may not be accessible in a flow device. A colorimetric resonant biosensor or a grating-based waveguide device operated in the static mode would be better suited and could provide a real time measurement of the progress of the attachment and folding.

One embodiment of this invention is to allow the measurement of binding and immobilization events in a non-polar environment or partially non-polar environment on a colorimetric resonant biosensor and/or a grating-based waveguide biosensor. *See e.g*., Cunningham et al., "Colorimetric resonant reflection as a direct biochemical assay technique," Sensors and Actuators B, Volume 81, p. 316-328, Jan 5 2002; U.S. Pat. Publ. No. 2004/0091397; U.S. Pat. No. 6,958,131; U.S. Pat. No. 6,787,110; U.S. Pat. No. 5,738,825; U.S. Pat. No. 6,756,078. Colorimetric resonant biosensors and grating-based waveguide biosensors are not surface plasmon resonant biosensors.

A colorimetric resonant biosensor has a colorimetric resonant diffractive grating surface that is used as a surface binding platform. A guided mode resonant phenomenon is used to produce an optical structure that, when illuminated with white light, is designed to reflect only a single wavelength. When molecules are attached to the surface, the reflected wavelength (color) is shifted due to the change of the optical path of light that is coupled into the grating. By linking receptor molecules to the grating surface, complementary binding molecules can be detected without the use of any kind of fluorescent probe or particle label. The detection technique is capable of resolving changes of ~0.1 nm thickness of material binding, and can be performed with the grating surface either immersed in fluid or dried

The readout system consists of, for example, a white light lamp that illuminates a small spot of the grating at normal incidence through, *e.g*., a fiber optic probe, and a spectrometer that collects the reflected light through a second fiber, also at normal incidence. A single spectrometer reading is performed in several milliseconds, thus it is possible to quickly measure a large number of molecular interactions taking place in parallel upon a grating surface, and to monitor reaction kinetics in real time. A maxima in reflected wavelength or a minima in transmitted wavelength of light can be used to illuminate the biosensor and a shift in wavelength of the light can be detected. A reaction in a grating-base waveguide biosensor can be determined by detecting a change in refractive index of light used to illuminate the biosensor, wherein a change in refractive index indicated that a species has interacted with the non-polar molecules on the biosensor surface. This technology is useful in, for example, applications where large numbers of biomolecular interactions are measured in parallel, particularly when molecular labels will alter or inhibit the functionality of the molecules under study. High throughput screening of pharmaceutical compound libraries with protein targets, and microarray screening of protein-protein interactions for proteomics are examples of applications that require the sensitivity and throughput afforded by this approach. *See also,* U.S. Ser. No. 60/244,312, filed 10/30/00; U.S. Ser. No. 09/929,957, filed 8/15/01; U.S. Ser. No. 60/283,314, filed 4/12/01; U.S. Ser. No. 60/303,028, filed 7/3/01; U.S. Ser. No. 09/930,352, filed 8/15/01; U.S. Ser. No. 10/415,037, filed 10/23/01;U.S. Ser. No. 10/399,940, filed 1/16/04; U.S. Ser. No. 10/059, 060, filed 1/28/02; U.S. Ser. No. 10/058,626, filed 1/28/02; U.S. Ser. No. 10/201,818, filed 7/23/02; U.S. Ser. No. 10/237,641, filed 9/9/02; U.S. Ser. No. 10/180,374, filed 6/26/02; U.S. Ser. No. 10/227,908, filed 8/26/02; U.S. Ser. No. 10/233,730, filed 9/3/02; U.S. Ser. No. 10/201,878, filed 7/23/02; U.S. Ser. No. 10/180,647, filed 6/26/02; U.S. Ser. No. 10/196,058, filed 7/15/02; U.S. Ser. No. 10/253,846, filed 9/25/02; U.S. Ser. No. 10/667696, filed 9/22/03, all of which are incorporated herein by reference in their entirety.

A surface of a colorimetric resonant biosensor or a grating-based waveguide is a material having a high refractive index, e.g., titanium dioxide, titanium oxide, or titanium phosphate. Various surface chemistries are compatible with the assembly of a biosensor supporting the study of proteins requiring a lipid environment. See, e.g., U.S. Pat. No. 6,645,644 (organic phosphonate and inorganic phosphate coatings); U.S. Pat. No. 6,146,767 (self-assembled organic monolayers); Gawalt et al. (2001) Langmuir, Self-Assembly and Bonding of Alkanephosphonic Acids on the Native Oxide Surface of Titanium 17 (19), 5736-5738 (assembly of an alkanephosphonic acid from solution on the native oxide surface of titanium followed by gentle heating gives an alkane chain ordered film of the acid which is strongly surface-bound); Gawalt et al., (1999) Langmuir, Enhanced bonding of alkanephosphonic acids to oxidized titanium using surface-bound alkoxyzirconium complex interfaces 15:8929-8933; Wang et al. (1998) Advanced Material Photogeneration of highly amphiphilic TiO2 surfaces 10(2):135-138; Folkers et al. (1995) Langmuir Self-assembled monolayers of long-chain hydroxamic acids on the native oxides of metals 11:813-824). In the invention a biosensor surface is coated with silane. The biosensor may additionally be coated with a surfactant, block copolymers of polyethylene oxide and polypropylene oxide in the form of PEO(a)-PPO(b)-PEO(a), or combinations thereof.

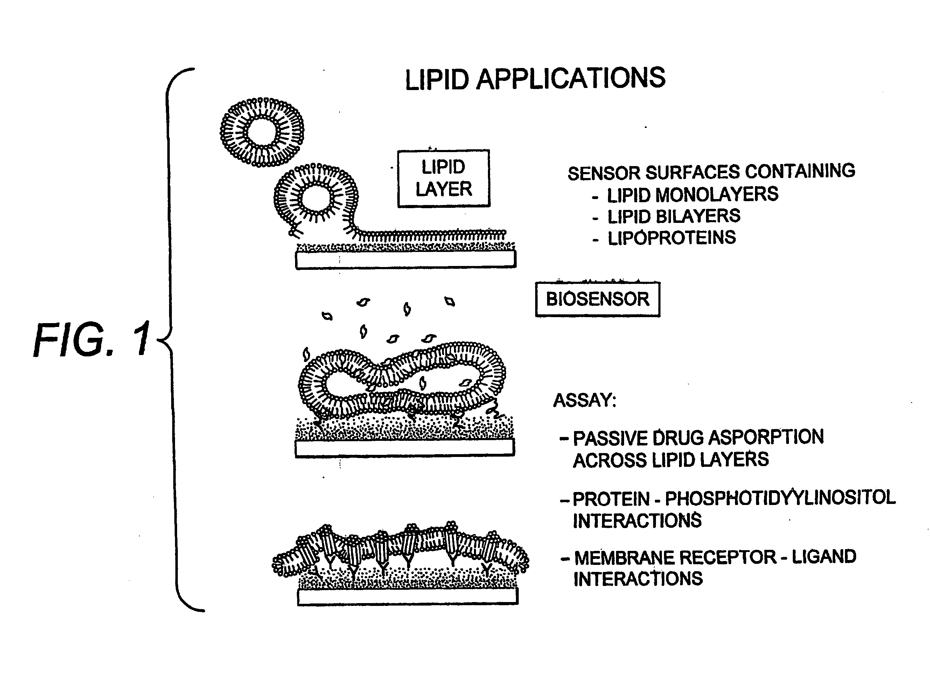

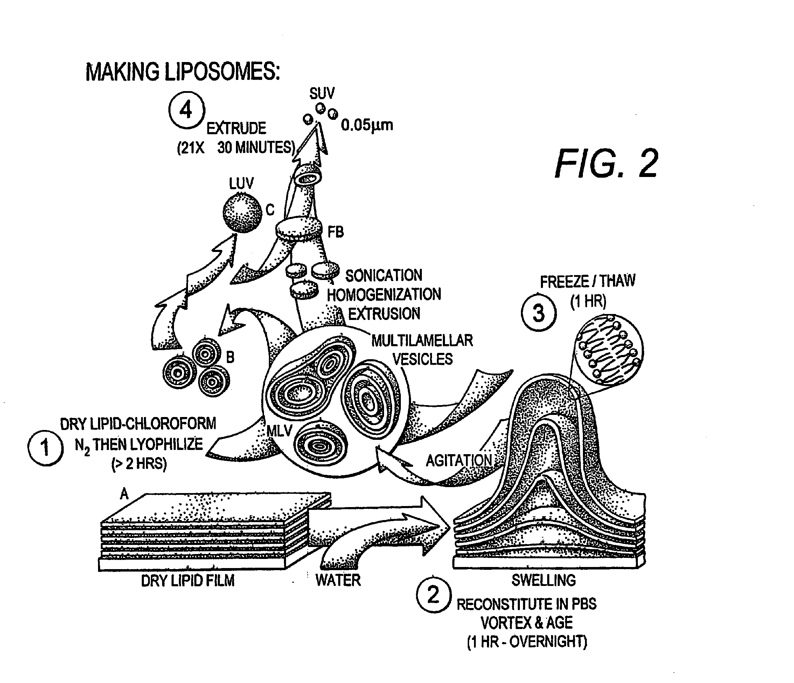

A TiO/TiO₂ coating of a colorimetric resonant biosensor is especially amenable to this application as the surface has an inherent hydrophobic character unless placed in a strong ultraviolet field or immersed in aqueous solutions for greater than 8 hrs. The treatment described herein make possible the study of binding events in a specialized biologically relevant environment for the measurement of binding events relevant to the study of cellular and life processes, especially without the use of labels in a microtiter or microchip format. Lipid monolayers and lipid bilayers can be immobilized to a biosensor surface of the invention. Once immobilized, they can be used to assay, for example, passive drug absorption across lipid layers, protein phosphotidylinositol interactions, and membrane receptor-ligand interactions by adding a species (i.e., any type of compound, lipid or protein) to the biosensor surface. See Figure 1 and Figure 2. In one embodiment of the invention, the non-polar molecules, the species, or both the non-polar molecules and the species are not labeled.

In particular, the invention makes possible the whole array of work related to the study of membrane bound proteins, especially the class known as G-Protein Couple Receptors, the most prevalent drug target by pharmaceutical companies and biotechnology companies. GPCRs can be immobilized to a colorimetric resonant biosensor surface or grating based waveguide biosensor in the context of, for example, a lipid membrane on the biosensor surface and can be used and to detect inhibitor binding. Another therapeutically important class of proteins related to this invention is ion channels and cell surface proteins that control intra- and intercellular signals. The array of work includes study of these proteins in their native environment as they interact with drugs, other membrane-bound or associated proteins, attached or not to cells, signal proteins, metabolites and undergo changes associated with these interactions such as inducement to interact with multiple components within the cell. Other non-polar materials can be immobilized on these surfaces for binding interaction analyses.

The functional advantages related to this invention include the combined properties of studying these proteins, lipids, lipid-based molecules, and other non-polar materials in their native environments without labels in, for example, microtiter wells. Binding interactions or structure changes can be quantified using the compositions and methods of the invention.

A lipid layer of any size or depth can be created on a colorimetric resonant biosensor surface, for example, a hydrophobic colorimetric resonant biosensor surface such at that described in Example 1. For example a lipid monolayer (e.g., POPC liposomes or micelles on TaO), lipid bilayer (e.g., hydrogel containing lipophilic groups (like alkanes) to anchor liposomes; Lahiri's amphiphilic anchor lipid surface which binds lipid bilayers (Langmuir 2000, 16, 7805-7810); biotinylated liposomes on a SA surface (Bieri et al., Nature Biotech. 1999 17, 1145-1108); Proteolipid bilayer (attach detergent solublized GPCR to surface in an oriented manner and reconstitute lipid membrane around immobilized GPCRs using lipid micelles while removing detergent (Analyt. Biochem. 2002 Jan 15;300(2):132-8; use membrane fractions containing over expressed receptors). Lipid surfaces can also be as described in, e.g., Baird et al., Analyt. Biochem. 2002 310:93-99; Stenlund et al., Analyt. Biochem. 2003, 316:243-250; Abdiche & Myszka, Analyt. Biochem. 2004 328:233-243; Ferguson et al., Bioconjugate Chem. 2005 16:1457-1483; U.S. Pat. No. 6,756,078.

The extent of lipid coverage on a colorimetric resonant biosensor or grating-based waveguide biosensor surface can be assayed by, for example, incorporating fluorescent lipids into liposomes and checking fluorescence signal, using a model predicted signal for lipid monolayer and bilayer; or monitoring BSA binding (a confluent lipid surface will resist BSA binding).

In one embodiment of the invention a biosensor of the invention can comprise an inner surface, for example, a bottom surface of a liquid-containing vessel. A liquid-containing vessel can be, for example, a microtiter plate well, a test tube, a petri dish, or a microfluidic channel. One embodiment of this invention is a biosensor that is incorporated into any type of microtiter plate. For example, a biosensor can be incorporated into the bottom surface of a microtiter plate by assembling the walls of the reaction vessels over the biosensor surface, so that each well can be exposed to a distinct test sample. Therefore, each individual microtiter plate well can act as a separate reaction vessel. Separate chemical reactions can, therefore, occur within adjacent wells without intermixing reaction fluids and chemically distinct test solutions can be applied to individual wells.

The most common assay formats for pharmaceutical high-throughput screening laboratories, molecular biology research laboratories, and diagnostic assay laboratories are microtiter plates. The plates are standard-sized plastic cartridges that can contain 96, 384, or 1536 individual reaction vessels arranged in a grid. Due to the standard mechanical configuration of these plates, liquid dispensing, robotic plate handling, and detection systems are designed to work with this common format. A biosensor of the invention can be incorporated into the bottom surface of a standard microtiter plate. Because the biosensor surface can be fabricated in large areas, and because the readout system does not make physical contact with the biosensor surface, an arbitrary number of individual biosensor areas can be defined that are only limited by the focus resolution of the illumination optics and the x-y stage that scans the illumination/detection probe across the biosensor surface. Each well of a microtiter plate can be, e.g., larger than about 1 mm² or about 5, 10, 15, 20, 30, 50, 100 mm² or larger.

In another embodiment of the invention a biosensor can be in a microarray format. One or more separate non-polar species (e.g., a lipid) "spots" are arranged in a microarray of distinct locations on a biosensor. A microarray of non-polar species spots comprises one or more non-polar species spots on a surface of a biosensor such that a surface contains many distinct locations, each with a different non-polar species spot or with a different amount of a non-polar species spot. For example, an array can comprise 1, 10, 100, 1,000, 10,000, or 100,000 distinct locations. Such a biosensor surface is called a microarray because one or more spots are typically laid out in a regular grid pattern in x-y coordinates. However, a microarray of the invention can comprise one or more spots laid out in any type of regular or irregular pattern. For example, distinct locations can define a microarray of spots of one or more specific non-polar species.

One example of a microarray of the invention is a nucleic acid microarray, in which each distinct location within the array contains a different nucleic acid molecule. In this embodiment, the spots within the nucleic acid microarray detect complementary chemical binding with an opposing strand of a nucleic acid in a test sample.

In one embodiment of the invention, about 100, 200, 300, 400, 500, 1,000, 2,000 or more individual wells or samples can be analyzed by one reader in about 5, 10, 20, 30,40, 50, 60 minutes or less.

The invention illustratively described herein suitably can be practiced in the absence of any element or elements, limitation or limitations that are not specifically disclosed herein. Thus, for example, in each instance herein any of the terms "comprising", "consisting essentially of", and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments, optional features, modification and variation of the concepts herein disclosed may be resorted to by those skilled in the art, and that such modifications and variations are considered to be within the scope of this invention as defined by the description and the appended claims.

In addition, where features or aspects of the invention are described in terms of Markush groups or other grouping of alternatives, those skilled in the art will recognize that the invention is also thereby described in terms of any individual member or subgroup of members of the Markush group or other group.

The following are provided for exemplification purposes only and are not intended to limit the scope of the invention described in broad terms above. All references cited in this disclosure are incorporated herein by reference.

### EXAMPLES

### Example 1: Stable Hydrophobic Biosensor Surfaces

A stable hydrophobic colorimetric resonant biosensor surface can be prepared by injecting repel silane (Amersham Biosciences 17-1332-01) into wells comprising a colorimetric resonant biosensor TiO surface, for example a 96-well plate. Incubate for 7 minutes. Inject 90ul hexane (anhydrous) (Sigma 227064) into each well. Aspirate 90ul of silane mixture with a multi-channel pipette. Inject 90ul hexane (2nd injection) into wells. Aspirate 90ul of silane mixture (2^{nd} aspiration) with a multi-channel pipette. Inject 90ul hexane (3rd injection) into wells. Aspirate all remaining solution in wells using an 8-channel stainless steel manifold connected to a vacuum pump. Allow the biosensor to cure in air after final aspiration. Wash the plate 2x with 100 µL PBS, then fill plate with 100 µL PBS. Sonicate the plate for 5-10 sec moving the plate back and forth in the sonication bath to disperse energy evenly across plate. Dry the backside of plate using the N₂ gun.

### Example 2: Hydrophobic Silane Sensor Treatment Poly-Phe-Lys Deposition Test for Hydrophobicity

**Table 1.**

| PPL | Plate 1 | | | Plate 2 | | | Plate 3 | | | Plate 4 | | | Plate 5 | | |
|---|---|---|---|---|---|---|---|---|---|---|---|---|---|---|---|
| | Shift | SD | %CV | Shift | SD | %CV | Shift | SD | %CV | Shift | SD | %CV | Shift | SD | %CV |
| NO | 4.0 | 0. 1 | 3.5 | 4.1 | 0. 1 | 3.4 | 4.0 | 0. 1 | 3.3 | 4.1 | 0. 1 | 3.3 | 4.1 | 0.1 | 2.9 |
| HEXANE | 4.2 | 0. 1 | 2.5 | 4.2 | 0. 1 | 2.9 | 4.0 | 0. 1 | 2.8 | 4.1 | 0. 1 | 2.5 | 4.1 | 0. 1 | 2.8 |
| REPEL | 4.8 | 0. 6 | 12.6 | 4.8 | 0. 6 | 12.1 | 4.6 | 0. 2 | 5.0 | 6.0 | 1.6 | 26.4 | 6.8 | 1.6 | 23.1 |

| | | | | | | | | | | | | | | | |
|---|---|---|---|---|---|---|---|---|---|---|---|---|---|---|---|
| **N for each reported number is 32 wells** | | | | | | | | | | | | | | | |

A colorimetric resonant biosensor surface was treated with repel silane (2% w/v in octamethylcyclotetrasiloxane) by incubating 50 µl of repel silane for a 7 minute treatment in the wells of a microtiter biosensor plate. The silane solution was aspirated and the surface washed 3x with 90 µl of hexanes. The microtiter biosensor plate was cured for 5 minutes after final hexanes wash by allowing the plate to dry. The plate was washed 3x with PBS pH 7.4. PPL was deposited onto the silane treated biosensor surface. 40 µl of 0.1 mg/ml PPL in 10 mM sodium phosphate pH 9.05 was added to each 6mm diameter well of the microtiter biosensor plate. The microtiter biosensor plate was dried overnight at RT. The plate was then washed 3x with PBS.

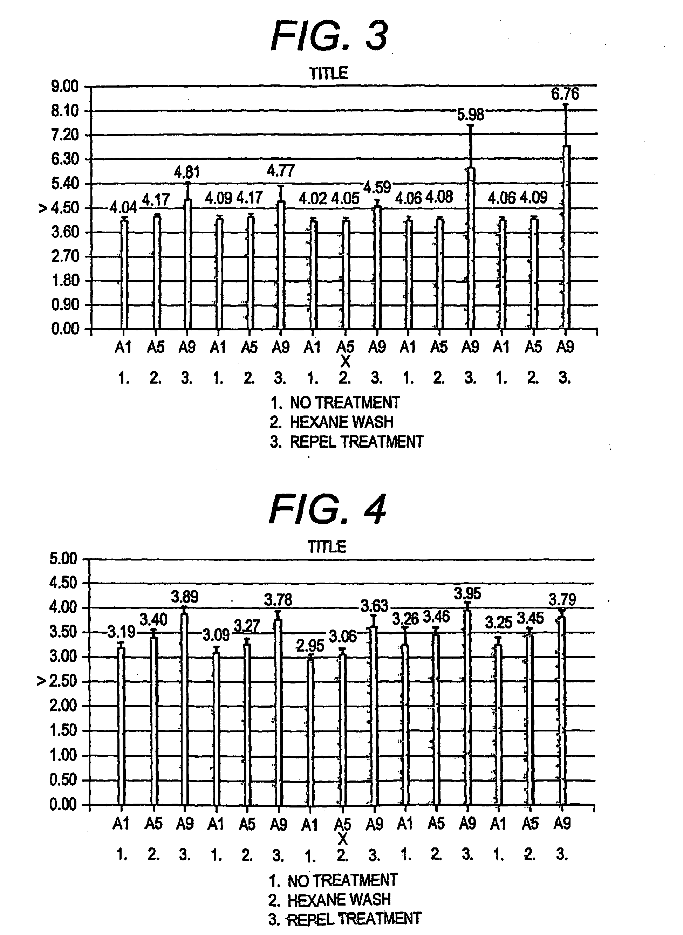

The treatment of the biosensor surface with repel silane increased the hydrophobic character of the biosensor surface as evidenced by the increasing attachment of poly-phe-lys to the TiO surface. Repel is a more effective hydrophobic surface treatment than a hexanes wash. *See,* Figure 3 and Table 1.

### Streptavidin Binding-Post PPL and aldehyde treatment

**Table 2.**

| SA | Plate 1 | | | Plate 2 | | | Plate 3 | | | Plate 4 | | | Plate 5 | | |
|---|---|---|---|---|---|---|---|---|---|---|---|---|---|---|---|
| | Shift | SD | %C | Shift | SD | %CV | Shift | SD | %CV | Shift | SD | %CV | Shift | SD | %CV |
| NO | 3.2 | 0.1 | 4.1 | 3.1 | 1 | 4.3 | 3.0 | 0. 1 | 4.6 | 3.3. | 0. 4 | 11.1 | 3.3. | 0.2 | 4.7 |
| HEXANE | 3.4 | 0. 2 | 5.1 | 3.3. | 0. 1 | 3.7 | 3.1 | 0. 1 | 4.5 | 3.5 | 0. 2 | 4.5 | 3.5 | 0. 1 | 4.3 |
| REPEL | 3.9 | 0. 1 | 3.8 | 3.8 | 0. 2 | 4.8 | 3.6 | 0. 2 | 6.1 | 3.9 | 0. 2 | 4.0 | 3.8 | 0. 2 | 4.4 |

| | | | | | | | | | | | | | | | |
|---|---|---|---|---|---|---|---|---|---|---|---|---|---|---|---|
| **N for each reported number is 32 wells** | | | | | | | | | | | | | | | |

The goal of a repel coating is to increase the hydrophobic character of TiO, a necessary property of a surface for the formation of biological membrane environment. Proper repel coating leads to an increase in adsorption to the biosensor of the test (protein) polymer poly phe-lys via non-polar interactions with the phenylalanine residues and the repel silane. The hydrophobic repel-type of surface is important for the creation of membrane-like environments on the biosensor that would support proper protein attachment and folding. In this case, the amines on the lysines (the amino acid residue in the polymer of phenyalanine-lysine not involved in the hydrophobic interaction) are subsequently converted chemically to aldehyde groups that can then form Schiff bases with other proteins (through their primary amines). In this example the protein is streptavidin.

50 µl of 0.1 mg/ml streptavidin in sodium acetate pH 5.0 was added to each 6mm diameter well of the microtiter biosensor plate. The solution was incubated for 1 hour in the well and followed with a 3 washes with sodium acetate pH 5.0. The results are shown in Figure 4 and Table 2. The data shown in Table 2 demonstrate that a uniform layer of a hydrophobic coating has been created on the biosensor, uniform chemical modification of this applied layer, and subsequent attachment of additional functional protein layers to the biosensor.
Poly-Phe-Lys vs. Poly-Lys Attachment Post Repel Silane Treatment

**Table 3.**

| **Polymer** | **Shift (nm)** | **SD** | **%CV** | **N** |
|---|---|---|---|---|
| PPL | 4.42 | 0.13 | 2.9 | 96 |
| PLL | 0.73 | 0.13 | 17.5 | 96 |
| PLL | 0.76 | 0.19 | 25.3 | 96 |
| PLL | 0.68 | 0.12 | 17.7 | 96 |
| PLL | 0.80 | 0.14 | 17.6 | 96 |

50 µl of repel silane was added to the wells of a biosensor microtiter plate for 7 minutes. The silane was aspirated and the biosensor was washed 3x with 90 µl of hexanes. The plate was cured for 5 minutes after the final hexanes wash. The plate was washed 3x with PBS pH 7.4. 40 µl 0.1 mg/ml PPL in 10 mM sodium phosphate pH 9.05 was added to the wells. The biosensor plate was dried overnight at RT and then washed 3x with PBS.

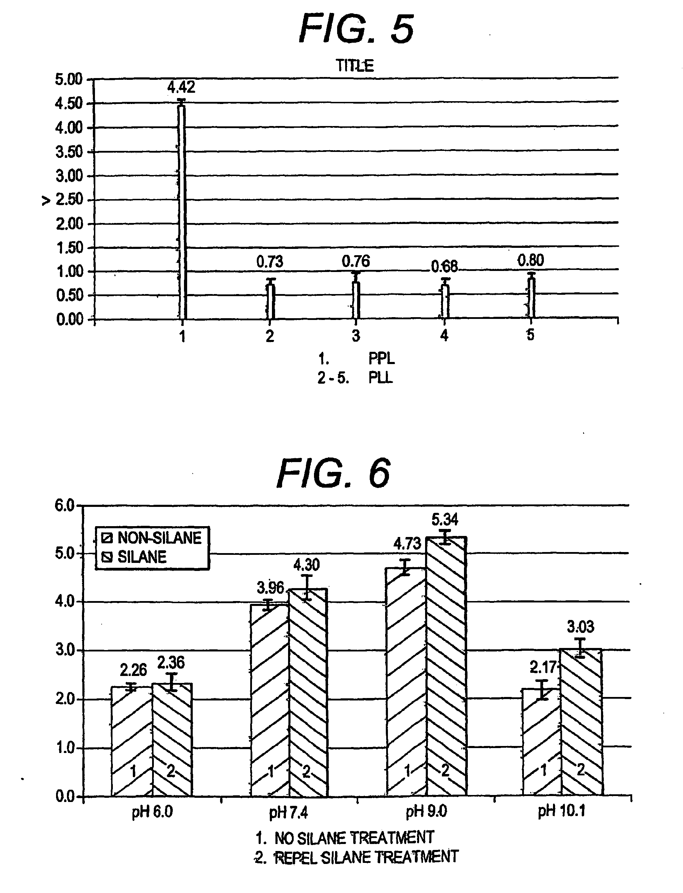

Figure 5 and Table 3 illustrate that binding is not driven by electrostatic interactions with the surface of the biosensor but rather by hydrophobic characteristics of biosensor surface. The two polymers poly-phe-lys (PPL) and poly-lys-lys (PLL) are nearly identical except for the presence of the hydrophobic phenylalanine residue in PPL. The lysine residue is positively charged and may affect binding of the polymers and is used to test this mode of attachment to the biosensor. In this example, clearly the charge on the lysines is not the major driving force for the attachment of the polymers to the biosensor as evidenced by its very low attachment as compared to the PPL. The biosensor is hydrophobically coated and attracts and adsorbs other hydrophobic polymers like this example protein (PPL).

### Example 3: Repel Silane Treatment to Improve PPL Binding

A biosensor plate was O₂ plasma treated prior to repel treatment. A 7 minute incubation of 50 µl repel silane treatment was performed in the 6mm diameter wells of biosensor plates. The remaining silane was aspirated and the biosensor was washed 3x with 90 µl of hexane. The biosensor plate was cured for 5 minutes after the final hexane wash. The biosensor plate was washed 3x with PBS pH 7.4. 0.1 mg/ml PPL solutions were prepared in 10 mM NaH₂PO₃ buffers at pHs of 6.0, 7.4, 9.0, and 10.1, these buffers were prepared by adjusting an 11 mM NaH₂PO₃ pH 4.0 buffer with 1 M NaOH. 40 ml of the PPL solutions were added to the wells of the biosensor plate and dried overnight at RT. The biosensor plate was washed 3x with PBS. Figure 6 shows the results of optimizing the hydrophobic coating to improve the capture of the model protein PPL.

### Example 4: Repel Silane Treatment to Increase Hydrophobic Character of Surface

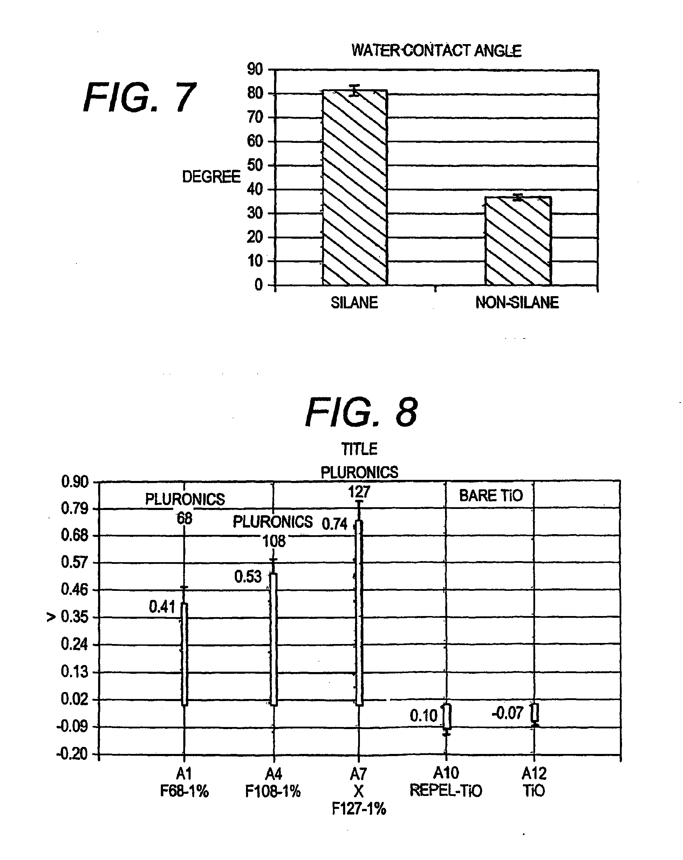

A biosensor was O₂ plasma treated prior to repel treatment. A 7 minute incubation of repel silane was performed on a section of biosensor material. The remaining silane was aspirated and the biosensor was washed 3x with hexane. The biosensor was cured for 5 minutes after the final hexane wash. The biosensor was then washed 3x with PBS pH 7.4. Water contact angle measurement was taken with an AST Product (Billerica, MA) Water Contact Angle Measurement Instrument. See, Figure 7.

The results of the water contact angle test further demonstrate that a hydrophobic surface is created on the biosensor surface. A higher water contact angle is indicative of a more hydrophobic surface. This type of test, confirming the creation of different layers on the biosensor surface for the preparation of lipid studies, is easily performed on a colorimetric resonant biosensor and is much more difficult to perform on the commercially available SPR flow devices owing to their very small surface area (typically~1mm²) and sealed nature of the provided flow cell. Because the current invention is generally on the order of ~28 mm² surface area and quite open, measurements of this type and other quantitative surface analyses (i.e. X-Ray Photo-electron Spectroscopy (XPS) Energy Dispersive Spectroscopy (EDS) Atomic Force Microscopy (AFM)) are easily accomplished.

**Table 4. PLURONIC® surfactants are block copolymers of polyethylene oxide and polypropylene oxide in the form of PEO(a)-PPO(b)-PEO(a).**

| Pluoronic type | a-b | Molecular weight of a-b, Da |
|---|---|---|
| F68F | 80-27 | 7680-9510 |
| F108NF | 141-44 | 12700-17400 |
| F127NF | 101-56 | 9840-14600 |

### PLURONIC® modification of TiO surface:

A1% solution of PLUROINIC® surfactants was made in water. A 100 µL of this solution was dispensed into bare TiO or hydrophobic TiO (repel silane modified) wells. The surfactant was allowed to adsorb to the surface of the sensor for 2 hrs. The biosensor was then washed 3x with water.

### Protein binding to biosensor surface

A streptavidin solution in PBS at 0.1 mg/mL was made. 100 µL of the solution was dispensed into the wells. The solution in the wells was incubated for 1 hr. The unbound material was removed and the biosensor was washed 3x with PBS.

### Creating surfaces with different protein density

Various concentrations of PLURONIC® F127 were made in water from 1 mg/mL to 0.001 mg/mL. 100 µL of PLURONIC® solution was dispensed into hydrophobic treated TiO (repel silane modified) wells. The surfactant was allowed to adsorb to the surface of the biosensor for 2 hrs. The liquid surfactant was removed and the biosensor was washed 3x with water. 100 µL of streptavidin solution (1mg/mL in PBS) was dispensed into PLURONIC® coated wells and incubated for 1 hour. The streptavidin was removed and the biosensor was washed 3x with PBS.

PLURONIC® surfactants are non-ionic detergents that form micelles in the same way that biological membranes create partitions in cellular environments, separating one type of molecular species such as non-polar moieties from polar moieties. This makes them appropriate models and reagents for creating environments on the biosensor that will allow the proper attachment and folding of mixed polar proteins (i.e., proteins with some polar regions and some non-polar regions).

### PLURONIC® binding to repel coated TiO

Figure 8 demonstrates higher amounts of PLURONIC® surfactant binding due to favorable interaction between the hydrophobic block of the surfactant (polypropylene oxide) to the hydrophobic repel surface. N=24 for PLURONIC® wells and N=8 for control wells.

### 1. PLURONIC® biosensors show reduced binding of proteins to TiO

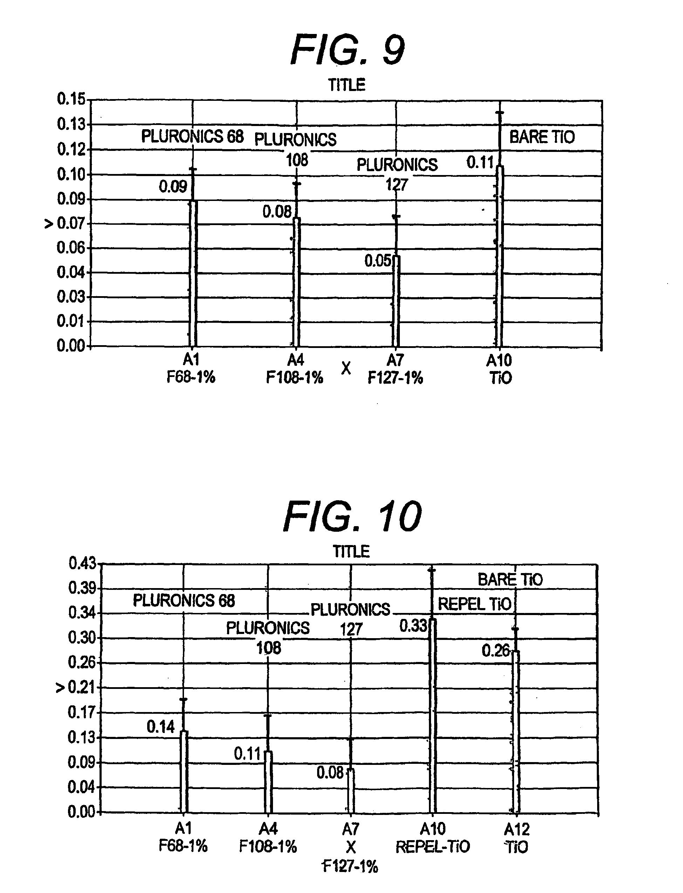

Figure 9 shows the reduction in streptavidin binding to bare TiO as a function of molecular weight of the PLURONIC® surfactants. This is a demonstration of the efficacy of the adsorption of the PLURONIC® detergent to the biosensor and the formation of a PLURONIC® layer on top of the repel layer, thus forming a bilayer (*i.e*., repel hydrophobic layer and PLURONIC® hydrophilic layer) on the biosensor. This is further demonstration of the ability to control the attachment and density of attachment of proteins to the bilayer modified sensor surface. N=24 for PLURONIC® wells and N=8 for control wells.

### 2. PLURONIC® surfactants reduce binding of streptavidin to TiO and repel-coated TiO

Figure 10 shows that a higher density of PLURONIC® adsorbed surface reduces streptavidin binding to the underlying hydrophobic TiO surface. Control surfaces include bare TiO and repel modified TiO. N=24 for PLURONIC® wells and N=8 for control wells.

### 3. PLURONIC® adsorbed surfaces used to create different densities of streptavidin by varying the concentration of the PLURONIC® layer concentration

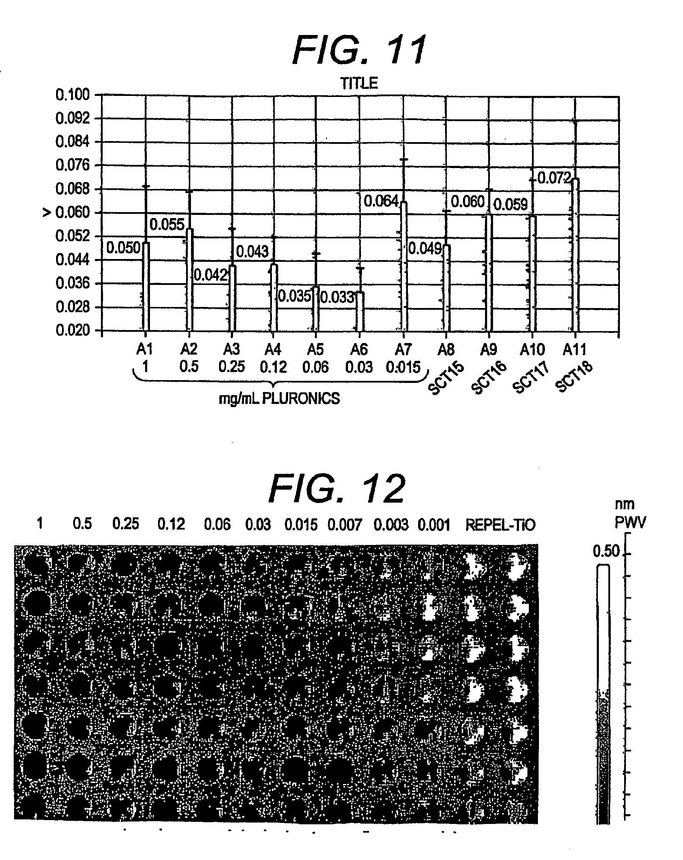

PLURONIC® surfactants can be used to control the amount of protein binding to the repel modified surfaces. The adsorbed PLURONIC® layer with dispersed proteins in it would be used as a model system for membrane bound proteins by properly selecting the proteins of interest and by carefully manipulating the density of the PLURONIC® layer to fit the protein in interstices in the adsorbed surfactant layer. Figure 11 shows streptavidin binding to various PLURONIC® modified surfaces. The amount of protein bound reduces with decreasing PLURONIC® density to a certain critical concentration and then increases as a function of the completeness/continuity of the PLURONIC®-coating.

The BIND Imager ^{™} image in Figure 12 shows the wells with different surface densities of streptavidin on PLURONIC® coated surfaces.

### Detergent TWEEN® 20 Binding

A biosensor plate was washed 3x with PBS pH 7.4. The PBS was removed and new solutions were added and incubated for 25 - 40 minutes:
a. PBS
b. 2.5% DMSO in PBS
c. 2.5% glycerol in PBS
d. 0.5% TWEEN® 20 in PBS
e. 5% DMSO in PBS

The plate was washed 10x with PBS.

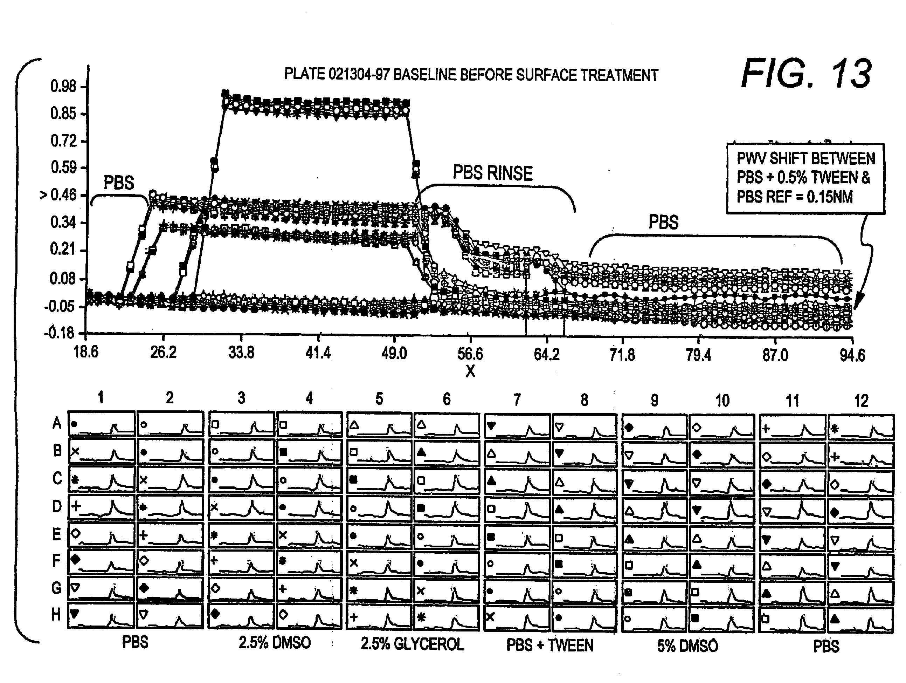

Detergents are important reagents in the composition of synthetic membrane-like environments. Figure 13 shows that a biosensor surface can be assembled with stable detergent absorption. TWEEN® is a non-ionic detergent with a polar end and a non-polar tail. The biosensor surface chemistry can be manipulated to orient TWEEN® as needed in the construction of a membrane-like environment.

### Modification of TiO2 surface with phosphoric acid to increase the density of silane layer

Phosphate containing environments are an important characteristic of biological lipids. They are generally found as a hetero-functional element of the lipid, sequestering and orienting the lipid so as to segregate non-polar compartments. This example demonstrates possession of the combined components of a biosensor and a membrane-like environment, especially as it relates to a lipid study supporting surface, a bilayer construction of a phosphate with a silane functionality.

### Protocol for modification of TiO2 surface to yield charged and reactive Titanium Phosphate (TiP) layer

TiO₂ coated BIND BIOSENSOR® samples were immersed in a 0.0145M H₃PO₄ solution in water for various time periods. Biosensor samples were removed from phosphoric acid bath and washed thoroughly with water. The samples were dried at 80°C for 18h and stored until further analysis and modification. A1% solution of aminopropyltrimethoxy silane was freshly made in 95% ethanol. Bioensor strips were immersed in a freshly made aminopropyltrimethoxy silane solution in 95% ethanol for 1 min, washed 4x with ethanol and dried under nitrogen. The biosensor strips were stored at 65% relative humidity overnight in A1 foil packages. Table 5 shows the XPS results of TiP and silane modified TiP surfaces.

**Table 5.**

| **Surface** | **Ti** | **C** | **N** | **O** | **Si** | **P** |
|---|---|---|---|---|---|---|
| TiO | 12.8 | 29.0 | 6.5 | 45.0 | 6.7 | 0 |
| TiO/SiO2 | 0.0 | 30.0 | 6.6 | 42.3 | 21.2 | 0.0 |
| H₃PO₄/15min | 7.6 | 30.9 | 4.9 | 46.6 | 5.4 | 4.6 |
| H₃PO₄/30min | 7.4 | 30.4 | 6.6 | 45.7 | 5.9 | 4.0 |
| H₃PO₄/1h | 9.6 | 24.3 | 5.3 | 51.2 | 5.6 | 4.1 |
| H₃PO₄/2h | 7.9 | 29.4 | 6.7 | 47.0 | 7.0 | 2.1 |

The Si column indicates the increase in silane content as a function of TiP modification with the highest silane content for a surface modified with H₃PO₄ for 2h. Silane and phosphate are clearly present. The data in Table 6 shows the atomic percentages from XPS results normalized without taking into account phosphorous and titanium and considering the contribution of silane alone in the XPS spectrum.

**Table 6 3-AMINOPROPYLTRIMETHOXY SILANE**

| **Elements** | **Stoichiometry** | **Relative %** | **TiO** | **H₃PO₄/15min** | **H₃PO₄/30min** | **H₃PC₄/30min** | **H₃PO₄/2h** |
|---|---|---|---|---|---|---|---|
| **C** | 3.0 | 37.5 | 29.0 | 30.9 | 30.4 | 24.3 | 29.4 |
| **O** | 3.0 | 37.5 | 45.0 | 46.6 | 45.7 | 51.2 | 47.0 |
| **N** | 1.0 | 12.5 | 6.5 | 4.9 | 6.6 | 5.3 | 6.7 |
| **Si** | 1.0 | 12.5 | 6.7 | 5.4 | 5.9 | 5.6 | 7.0 |
| **Total** | 8.0 | 100.0 | 87.2 | 87.8 | 88.7 | 86.4 | 90.1 |

| **Normalized without Phosphorus and Titanium** | | | | | | | |
|---|---|---|---|---|---|---|---|
| **Elements** | **Stoichiometry** | **Relative %** | **TiO** | **H₃PO₄/15min,** | **H₃PO₄/30min** | **H₃PO₄/1h** | **H₃PO₄/2h** |
| **C** | 3.0 | 37.5 | 33.3 | 35.2 | 34.4 | 28.2 | 32.6 |
| **O** | 3.0 | 37.5 | 51.7 | 53.1 | 51.6 | 59.4 | 52.2 |
| **N** | 1.0 | 12.5 | 7.5 | 5.6 | 7.5 | 6.1 | 7.4 |
| **Si** | 1.0 | 12.5 | 7.7 | 6.1 | 6.7 | 6.5 | 7.8 |
| **Total** | 8.0 | 100.0 | 100.3 | 100.0 | 100.2 | 100.2 | 100.0 |
| | | Norm.Fact= | 1.1 | 1.1 | 1.1 | 1.2 | 1.1 |

Although the silane content of H₃PO₄ modified surface is comparable to the TiO surface, the activity of the amine group in the two surfaces is different as evidenced by measuring aldehyde reaction extent of amino-silane modified surfaces using glutaraldehyde modification reagent. The significant presence of nitrogen (N) and silicon (Si) shown in Table 6 demonstrate the stable application and modification by the intended material(s), 3-aminopropyltrimethoxysilane, to the biosensor surface. In fact, the presence of these two elements was not expected at the surface depth analyzed, except by the stable addition of the intended material.

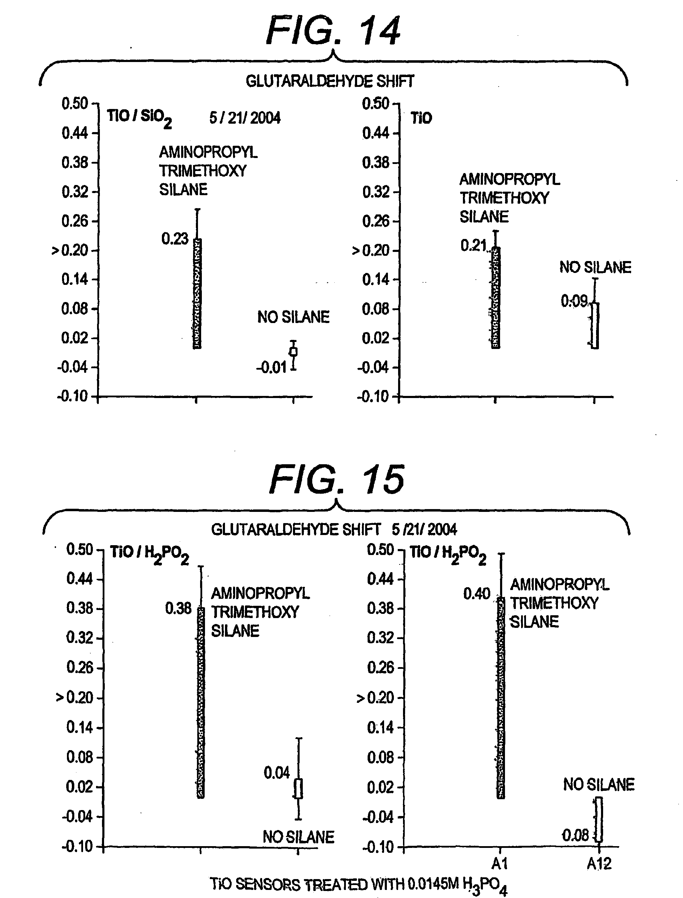

Figure 14 shows a BIND BIOSENSOR® signal for aldehyde modification of a silane modified TiO and a TiO/SiO2 surface. N=1 plate (96 wells).

Figure 15 shows an improvement in aldehyde modification as evidenced by higher BIND BIOSENSOR® PWV shift for TIP modified silane surfaces. The results are repeatable. N=1 plate (96 wells).

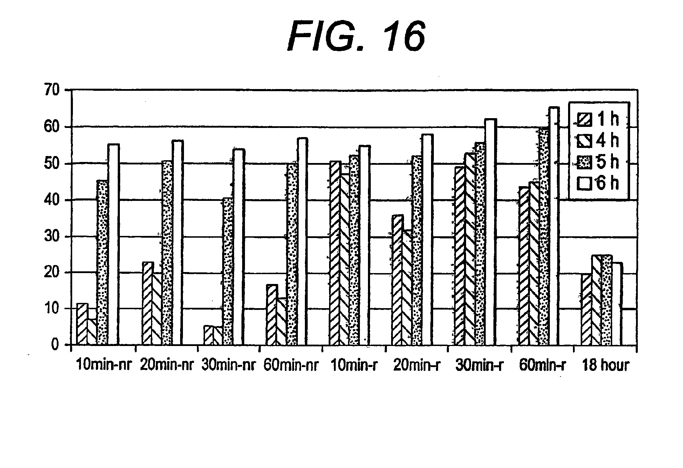

Figure 16 shows the permanency of the TiP layer when the samples treated with phosphoric acid were dried at 18h at 80°C. Samples dried at RT are compared using water contact angle results as a function of time. The annealed samples maintain a hydrophilic surface as evidenced by stable low contact angles over time while un-annealed samples revert back to high contact angles (~60-70) characteristic of native TiO2 surface.

We have demonstrated the creation of various mono and bilayers that could control the attachment of membrane proteins. To one skilled in the art of using alternative reagents such as different hydrophobic materials including but not limited to lipids, hetero and homo - functional lipids, phospholipids, cholesterol, single and double-chain amphiphiles, micelle forming compounds, liposome forming materials, ionic detergents, anionic detergents, cationic detergents, zwitter-ionic detergents and other reagents as needed to create appropriate environments for enabling the attachment and folding of membrane bound proteins, the present invention becomes a tool for the study of the attachment, folding, and binding of biological molecules, small molecules, and test reagents either directly or to previously immobilized proteins to a biosensor in said membraneous environment

## Claims

1. A colorimetric resonant biosensor or a grating-based waveguide biosensor, wherein the surface of the biosensor is titanium oxide, titanium dioxide, or titanium phosphate, wherein the titanium oxide, titanium dioxide, or titanium phosphate surface is coated with silane to form a titanium oxide-silane, titanium dioxide-silane, or titanium phosphate-silane surface; and wherein a lipid bilayer or a lipid monolayer is immobilized on the titanium oxide-silane, titanium dioxide-silane, or titanium phosphate-silane surface.

2. The biosensor of claim 1, wherein the biosensor is incorporated into the bottom of a microtiter plate or is in a microarray format.

3. The biosensor of claim 2, wherein the biosensor is incorporated into the bottom of a microtiter plate, and wherein each well of the microtiter plate is about 5 mm² to about 50 mm².

4. The biosensor of claim 1, wherein the lipid bilayer or lipid monolayer has no label.

5. The biosensor of claim 1, wherein the biosensor is further coated with one or more surfactants.

6. The biosensor of claim 1, wherein the titanium oxide-silane surface, titanium dioxide-silane surface, or titanium phosphate-silane surface is coated with block copolymers of polyethylene oxide and polypropylene oxide in the form of PEO(a)-PPO(b)-PEO(a).

7. A method of analyzing a chemical or physical interaction in a lipid bilayer or a lipid monolayer, using a colorimetric resonant biosensor or a grating-based waveguide biosensor, wherein the biosensor comprises a titanium oxide-silane, titanium dioxide-silane, or titanium phosphate-silane surface, and wherein a lipid bilayer or a lipid monolayer is immobilized on the titanium oxide-silane, titanium dioxide-silane, or titanium phosphate-silane surface, the method comprising: contacting the lipid layer with a species and analyzing the interaction of the lipid layer and the species by (a) detecting a maxima in reflected wavelength or a minima in transmitted wavelength of light used to illuminate the biosensor, wherein if the wavelength of light is shifted the species has interacted with the lipid layer; or (b) detecting a change in refractive index of light used to illuminate the biosensor, wherein a change in refractive index indicated that the species has interacted with the lipid layer.

8. The method of claim 7, wherein the biosensor is incorporated into the bottom of a microliter plate or is in a microarray format.

9. The method of claim 7, wherein the biosensor is incorporated into the bottom of a microtiter plate, and wherein each well of the microtiter plate is about 5 to about 50 mm².

10. The method of claim 7, wherein about 300 or more samples can be analyzed in about ten minutes or less.

11. The method of claim 7, wherein the lipid layer with is contacted with a species under static conditions.

12. The method of claim 7, wherein the interaction of the lipid layer and the species is analyzed under static conditions.

13. The method of claim 7, wherein the lipid layer and the species arc label-free.

## Patentansprüche

1. Colorimetrischer resonanter Biosensor oder gitterbasierter Wellenleiter-Biosensor, wobei die Oberfläche des Biosensors aus Titanoxid, Titandioxid oder Titanphosphat ist, wobei die Titanoxid-, Titandioxid- oder Titanphosphatoberfläche unter Ausbildung einer Titanoxid-Silan-, Titandioxid-Silan- oder Titanphosphat-Silan-Oberfläche mit Silan beschichtet ist; wobei eine Lipid-Doppelschicht oder eine Lipid-Einzelschicht auf der Titanoxid-Silan-, Titandioxid-Silan- oder Titanphosphat-Silan-Oberfläche immobilisiert ist.

2. Biosensor nach Anspruch 1, wobei der Biosensor in den Boden einer Mikrotiterplatte eingebaut ist oder in einem Mikroarrayformat vorliegt.

3. Biosensor nach Anspruch 2, wobei der Biosensor in den Boden einer Mikrotiterplatte eingebaut ist und wobei jedes Well der Mikrotiterplatte etwa 5 mm² bis etwa 50 mm² groß ist.

4. Biosensor nach Anspruch 1, wobei die Lipid-Doppelschicht oder Lipid-Einzelschicht keine Markierung aufweist.

5. Biosensor nach Anspruch 1, wobei der Biosensor weiterhin mit einem oder mehreren Tensiden beschichtet ist.

6. Biosensor nach Anspruch 1, wobei die Titanoxid-Silan-, Titandioxid-Silan- oder Titanphosphat-Silan-Oberfläche mit einem Block-Copolymer aus Polyethylenoxid und Polypropylenoxide in der Form PEO(a)-PPO(b)-PEO(a) beschichtet ist.

7. Verfahren zur Analyse einer chemischen oder physikalischen Wechselwirkung in einer Lipid-Doppelschicht oder einer Lipid-Einzelschicht unter Verwendung eines colorimetrischen resonanten Biosensors oder gitterbasierten Wellenleiter-Biosensors, wobei der Biosensor eine Titanoxid-Silan-, Titandioxid-Silan- oder Titanphosphat-Silan-Oberfläche umfasst und wobei eine Lipid-Doppelschicht oder eine Lipid-Einzelschicht auf der Titanoxid-Silan-, Titandioxid-Silan- oder Titanphosphat-Silan-Oberfläche immobilisiert ist, wobei das Verfahren umfasst: In-Kontakt-bringen der Lipidschicht mit einer Spezies und Analysieren der Wechselwirkung der Lipidschicht und der Spezies durch (a) Erfassen eines Maximums einer reflektierten Wellenlänge oder eines Minimums einer übertragenen Wellenlänge von Licht, das zum Beleuchten des Biosensors verwendet wird, wobei die Spezies im Falle einer Verschiebung der Wellenlänge des Lichts mit der Lipidschicht in Wechselwirkung getreten ist; oder (b) Erfassen einer Veränderung des Brechungsindexes von Licht, das zum Beleuchten des Biosensors verwendet wird, wobei eine Veränderung des Brechungsindexes anzeigt, dass die Spezies mit der Lipidschicht in Wechselwirkung getreten ist.

8. Verfahren nach Anspruch 7, wobei der Biosensor in den Boden einer Mikrotiterplatte eingebaut ist oder in einem Mikroarrayformat vorliegt.

9. Verfahren nach Anspruch 7, wobei der Biosensor in den Boden einer Mikrotiterplatte eingebaut ist und wobei jedes Well der Mikrotiterplatte etwa 5 bis etwa 50 mm² groß ist.

10. Verfahren nach Anspruch 7, wobei etwa 300 oder mehr Proben in etwa zehn Minuten oder weniger analysiert werden können.

11. Verfahren nach Anspruch 7, wobei die Lipidschicht mit einer Spezies unter statischen Bedingungen in Kontakt ist.

12. Verfahren nach Anspruch 7, wobei die Wechselwirkung der Lipidschicht und der Spezies unter statischen Bedingungen analysiert wird.

13. Verfahren nach Anspruch 7, wobei die Lipidschicht und die Spezies markierungsfrei sind.

## Revendications

1. Biocapteur résonant colorimétrique ou biocapteur à guide d'ondes en réseau, dans lequel la surface du biocapteur est en oxyde de titane, en dioxyde de titane ou en phosphate de titane, dans lequel la surface en oxyde de titane, en dioxyde de titane ou en phosphate de titane est revêtue de silane afin de former une surface en oxyde de titane-silane, en dioxyde de titane-silane ou en phosphate de titane-silane, et dans lequel une bicouche lipidique ou une monocouche lipidique est immobilisée sur la surface en oxyde de titane-silane, en dioxyde de titane-silane ou en phosphate de titane-silane.

2. Le biocapteur de la revendication 1, dans lequel le biocapteur est incorporé au fond d'une plaque microtitre ou se présente sous la forme d'un microréseau.

3. Le biocapteur de la revendication 2, dans lequel le biocapteur est incorporé au fond d'une plaque microtitre, et dans lequel chaque puits de la plaque microtitre mesure d'environ 5 mm² à environ 50 mm².

4. Le biocapteur de la revendication 1, dans lequel la bicouche lipidique ou la monocouche lipidique ne porte pas de marqueur.

5. Le biocapteur de la revendication 1, dans lequel le biocapteur est également revêtu d'un ou de plusieurs tensioactifs.

6. Le biocapteur de la revendication 1, dans lequel la surface en oxyde de titane-silane, la surface en dioxyde de titane-silane ou la surface en phosphate de titane-silane est revêtue de copolymères en bloc d'oxyde de polyéthylène et d'oxyde de polypropylène sous la forme PEO(a)-PPO(b)-PEO(a).

7. Méthode d'analyse d'une interaction chimique ou physique dans une bicouche lipidique ou une monocouche lipidique, utilisant un biocapteur résonant colorimétrique ou un biocapteur à guide d'ondes en réseau, dans laquelle le biocapteur comprend une surface en oxyde de titane-silane, en dioxyde de titane-silane ou en phosphate de titane-silane, et dans laquelle une bicouche lipidique ou une monocouche lipidique est immobilisée sur la surface en oxyde de titane-silane, en dioxyde de titane-silane ou en phosphate de titane-silane, la méthode comprenant les étapes suivantes : mise en contact de la couche lipidique avec une espèce et analyse de l'interaction entre la couche lipidique et l'espèce par (a) détection d'une longueur d'onde réfléchie maximum ou d'une longueur d'onde transmise minimum de la lumière utilisée pour éclairer le biocapteur, un décalage de la longueur d'onde de la lumière indiquant que l'espèce a interagi avec la couche lipidique ; ou (b) détection d'une modification de l'indice de réfraction de la lumière utilisée pour éclairer le biocapteur, une modification de l'indice de réfraction indiquant que l'espèce a interagi avec la couche lipidique.

8. La méthode de la revendication 7, dans laquelle le biocapteur est incorporé au fond d'une plaque microtitre ou se présente sous la forme d'un microréseau.

9. La méthode de la revendication 7, dans laquelle le biocapteur est incorporé au fond d'une plaque microtitre, et dans laquelle chaque puits de la plaque microtitre mesure d'environ 5 mm² à environ 50 mm².

10. La méthode la revendication 7, dans laquelle un minimum de 300 échantillons environ peuvent être analysés en un maximum de dix minutes environ.

11. La méthode la revendication 7, dans laquelle la couche lipidique est mise en contact avec une espèce dans des conditions statiques.

12. La méthode la revendication 7, dans laquelle l'interaction entre la couche lipidique et l'espèce est analysée dans des conditions statiques.

13. La méthode la revendication 7, dans laquelle la couche lipidique et l'espèce ne portent pas de marqueur.
